# EUROPEAN PATENT SPECIFICATION

(11) **EP 0 231 093 B1**
(45) Date of publication and mention of the grant of the patent: **12.01.1994**
(21) Application number: 87300458.4
(22) Date of filing: 20.01.1987
(51) Int. Cl.: C12N 11/08, C08G 77/14

(54) **An organopolysiloxane gel and an immobilized enzyme supported thereon**
Organopolysiloxangel und ein darauf immobilisiertes Enzym
Gel d'organopolysiloxane et une enzyme immobilisée sur ce gel

(30) Priority: 20.01.1986 JP 9653/86
(43) Date of publication of application: 05.08.1987
(73) Proprietor: Shin-Etsu Chemical Co., Ltd., Chiyoda-ku Tokyo 100 (JP)
(72) Inventor: Yamaya, Masaaki, Annaka-shi Gunma-ken (JP); Kanbara,Hiroshi, Annaka-shi Gunma-ken (JP)
(74) Representative: Baverstock, Michael George Douglas

(56) References cited:
- EP-A- 0 097 364
- DE-A- 1 936 067
- FR-A- 2 142 950
- FR-A- 2 322 155
- FR-A- 2 558 171

## Description

The present invention relates to an organopolysiloxane gel, especially such a gel suitable as a carrier for the immobilization of an enzyme, such as aminoacylase, trypsin, glucoamylase and the like, by covalent bonding as well as an immobilized enzyme supported on the gel as a carrier.

As is well known, it is a trend in recent years that various enzymatic reactions are performed by using an immobilized enzyme in view of the advantage in respect of the high productivity of the process in comparison with the use of a free enzyme. Such an immobilized enzyme can be prepared in the prior art by various methods differing in the principle of immobilization without or with the use of a carrier, including the crosslinking method in which the molecules of the enzyme per se are crosslinked by using a crosslinking agent, the inclusion method in which the molecules of the enzyme are enclosed within a matrix material as a carrier and the carrier-bonding method in which the enzyme molecules are bonded to the carrier. These prior art methods have their respective problems and disadvantages. For example, the crosslinking method is disadvantageous in respect of the relatively low efficiency of immobilization. The inclusion method has a problem that the molecules of the enzyme sometimes fall off the matrix when the matrix material is swollen in the course of the enzymatic process because no bonding is formed between the enzyme molecules and the matrix.

The carrier-bonding method is also classified into three types depending on the nature of the bonding between the enzyme molecules and the carrier,including the physical adsorption method, ionic bonding method and covalent bonding method. A problem common in the physical adsorption and ionic bonding methods is the relatively low bonding force between the enzyme molecules and the carrier to cause falling of the enzyme molecules off the carrier. In this regard, the covalent bonding method is generally preferred in the absence of a gradual decrease of the enzymatic activity in the lapse of time.

Various attempts and proposals have been made in the prior art for the immobilization of an enzyme by the covalent bonding method For example EP-A-97 364. Japanese Patent Publication 55-32375 teaches a method of enzyme immobilization using a silane coupling agent. The immobilization in this case is performed by the Schiff base method in which the enzyme can be immobilized under relatively mild conditions little affecting the activity of the enzyme. In addition, the enzyme molecules immobilized by this method are firmly bonded to the carrier so that the immobilized enzyme on the carrier is resistant against swelling in water or an organic solvent and little influenced by temperature increase or pressure and the enzymatic activity thereof is free from the problem of decrease with time because the enzyme molecules are persistently retained on the carrier. On the other hand, this method of using a silane coupling agent is disadvantageous in respect of the lengthy process including a number of steps as well as the relatively low efficiency of enzyme immobilization. This problem of low efficiency of immobilization is particularly serious when the silane coupling agent is an amino-containing one such as 3-aminopropyl triethoxy silane because hydrogen bonding takes place between the amino groups in the silane compound and the hydroxy groups on the carrier surface preferentially to bonding between hydrolyzable ethoxy groups of the silane compound and the hydroxy groups on the carrier, thus decreasing the amount of the functional amino groups available for the immobilization of the enzyme so that the efficiency of immobilization is unavoidably low.

Alternatively, Japanese Patent Kokai 52-56092 teaches the use of a silane coupling agent which is an organosilane compound having an aldehydo group -CHO or an acetal structure, i.e. gem-dialkoxy-substituted carbon atom, in the substituent to the silicon atom in view of the high reactivity of these groups or structures with the amino-containing residue of the enzyme. This method is also not free from the problem accompanying the lengthy process for the enzyme immobilization including a number of steps so that the percentage of immobilized enzyme can rarely exceed 30 to 40% as a result of the incompleteness in the treatment of the carrier material with the silane coupling agent

The invention provides a method of preparing an organopolysiloxane gel of the chemical composition represented by the average unit formula 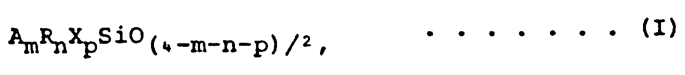
in which A is a monovalent hydrocarbon group having at least one aldehydo group -CHO or an acetal group -CH(OR¹)₂, R¹ being an alkyl group, R is a monovalent hydrocarbon group having 1 to 18 carbon atoms, X is a hydroxy group or a silicon-bonded hydrolyzable group, e.g. alkoxy groups, the subscript m is a positive number in the range from 0.01 to 1, the subscript n is zero or a positive number smaller than 1 with the proviso that m+n is a positive number not exceeding 1 and the subscript p is a positive number smaller than 0.5.

Accordingly, the immobilized enzyme comprises:
(a) a carrier material which is an organopolysiloxane gel as defined above; and
(b) an enzyme covalently bonded to the carrier material.

As is understood from the above given summary of the invention, the most characteristic feature of the claimed method consists in the use of a very specific carrier material which is an organopolysiloxane gel represented by the average unit formula (I) defined above. By virtue of the unique chemical structure of the carrier material, in particular, having the aldehydo groups or acetal groups reactive with the amino groups in the enzyme molecules, the enzyme can easily be immobilized in an aqueous medium under mild conditions of, for example, room temperature and neutrality of the medium in an unexpectedly high efficiency of 60 to 80% as a consequence of the high density of the functional groups reactive with the enzymatic amino groups.

Further, the organopolysiloxane gel as the carrier material can readily be prepared in one step by (co)hydrolyzing an organosilane compound having the functional group denoted by the symbol A in the average unit formula (I) and hydrolyzable groups alone or as a mixture with one or more of other hydrolyzable organosilane compounds.

In the average unit formula (I), the symbol A denotes a monovalent hydrocarbon group having at least one aldehydro group -CHO or gem-dialkoxy-substituted carbon atom -CH(OR¹)₂, R¹ generally being a lower alkyl group having 1 to 4 carbon atoms such as, preferably, methyl and ethyl. A is selected from groups expressed by the following formulae, in which the symbols Me, Et and Pr denote methyl, ethyl and isopropyl groups, respectively:
-CH₂CH₂CHO;
-CHMeCH₂CHO;
-CH₂CH₂CH(OMe)₂;
-CH₂CH₂CH(OEt)₂;
-CH₂CH(OEt)₂;
-CMe₂(CH₂)₃CHMeCH₂CHO;
-CMe₂(CH₂)₃CHMeCH(OMe)₂;
-CHPr(CH₂)₂CHMeCH₂CHO;
-CHPr(CH₂)₂CHMeCH₂CH(OMe)₂;
-(CH₂)₁₀-CHO; and
-(CH₂)₁₀-CH(OMe)₂.

Preferred are:
-CH₂CH₂CHO;
-CMe₂(CH₂)₃CHMeCH₂CHO; and
-CH₂CH₂CH(OEt)₂.

The symbol R in the formula (I) is a monovalent hydrocarbon group having 1 to 18 carbon atoms and selected generally from alkyl groups, e.g. methyl, ethyl, propyl, butyl, octyl and octadecyl groups, alkenyl groups, e.g. vinyl and allyl groups, aryl groups, e.g. phenyl and tolyl groups, and cycloalkyl groups, e.g. cyclohexyl group as well as substituted hydrocarbon groups obtained by replacing a part or all of the hydrogen atoms in these hydrocarbon groups with halogen atoms, cyano groups and the like such as chloromethyl, 3,3,3-trifluoropropyl and 2-cyanoethyl groups and other haloalkyl and cyanoalkyl groups. The group R is optional in the inventive method of preparing an organopolysiloxane gel since n may be zero.

The symbol X in the formula (I) is a silanolic hydroxy group or a silicon-bonded hydrolyzable group exemplified by alkoxy groups, e.g. methoxy, ethoxy, isopropoxy and butoxy groups, and acyloxy groups, e.g. acetoxy (or other lower alkoxy) and benzoyloxy groups.

The subscripts m, n and p each give the molar ratio of the respective groups A, R and X to the silicon atoms in the gel. An organopolysiloxane gel in which m is larger than 1 is not practical since difficulties are encountered in the preparation of such a gel by the (co)hydrolysis of a silane or a silane mixture corresponding to such a chemical composition. When n is larger than 1, such an organopolysiloxane is less hydrophilic and less dispersible in water so that the gel is less receptive of the enzyme dissolved in an aqueous medium. When p is 0.5 or larger, the enzyme would be bonded to the carrier gel by forming hydrogen bonds between the amino residues of the enzyme and the groups X in the carrier in an increased proportion with correspondingly decreased proportion of the enzyme molecules covalently bonded to the carrier gel. In this regard, m should be a positive number in the range from 0.01 to 1, n should be zero or a positive number smaller than 1 and p should be a positive number smaller than 0.5.

The organopolysiloxane gel represented by the average unit formula (I) can be prepared by hydrolyzing an organosilane compound having one or more of the silicon-bonded groups A and hydrolyzable group or groups in a molecule or cohydrolyzing a silane mixture composed of such an organosilane compound and other hydrolyzable organosilane compound. The organosilane compound having the silicon-bonded group A, i.e. monovalent hydrocarbon group having an aldehydo group or acetal group, can be synthetically prepared by the hydrosilation addition reaction between an organohydrogen silane, i.e. an organosilane having a hydrogen atom directly bonded to the silicon atom, and an aliphatically unsaturated aldehyde compound such as acrolein, crotonaldehyde, undecylenic aldehyde, citral, citronellal and the like or a compound acetalized therewith in the presence of a platinum catalyst such as chloroplatinic acid. The (co)hydrolysis is performed preferably in the presence of an inorganic acid such as hydrochloric, sulfuric, nitric or hydrofluoric acid although certain Lewis acids such as titanium chloride or aluminum chloride can be used instead. It is sometimes advantageous that a metal oxide such as zirconium oxide or aluminum oxide is added to the medium for the hydrolysis so that the resultant organopolysiloxane gel may contain such an oxide to be imparted with improved heat resistance and resistance against chemicals, e.g. acids.

The organopolysiloxane gel obtained in the above described manner is useful as a carrier for the immobilization of various kinds of enzymes, e.g. aminoacylase, trypsin, glucoamylase and the like, by forming covelent bonds therebetween. The treatment for forming the covalent bonds between them can be performed under mild conditions in a high efficiency. In addition, the organopolysiloxane gel can be used as a material for the stationary phase in various chromatographic processes such as liquid chromatography and affinity chromatography.

The above mentioned enzymes can be immobilized on the organopolysiloxane gel as a carrier in an aqueous medium adjusted to have the optimum pH of the respective enzyme without increasing the temperature although it is optional to use a non-aqueous medium, such as hydrocarbon solvents and chlorinated hydrocarbon solvents, at an elevated temperature as is taught in French Patent 7330413. The immobilized enzyme obtained in this manner is outstandingly stable and resistant against variations in various factors such as temperature, pH, pressure and so on to exhibit durable enzymatic activity.

In the following, the inventive method of preparing organopolysiloxane gels as well as immobilized enzymes supported on the gel as the carrier are described in more detail by way of examples.

### Example 1.

### [Preparation of an acetal-containing organopolysiloxane gel]

Into a reaction vessel of 300 ml capacity were introduced 31.5 g (0.242 mole) of acrolein diethyl acetal CH₂=CH-CH(OC₂H₅)₂, 10.4 mg (0.02 m mole) of chloroplatinic acid and 180 ml of toluene and the mixture was heated to 60 °C. Then, 44.3 g (0.363 mole) of trimethoxy silane HSi(OCH₃)₃ were gradually introduced into the mixture kept at the same temperature and the reaction mixture was further agitated at 100 °C for 2 hours after completion of the introduction of the silane compound. After cooling, the reaction mixture was subjected to distillation under reduced pressure to give 41.6 g of a fraction which could be identified to be 3,3-diethoxypropyl trimethoxy silane of the formula (CH₃O)₃SiCH₂CH₂CH(OC₂H₅)₂, which is referred to as the acetal silane hereinbelow. The yield was 0.165 mole corresponding to 68% of the theoretical value.

A mixture composed of 10 g (0.040 mole) of the acetal silane, 84 g (0.400 mole) of ethyl silicate and 68 g (1.50 moles) of ethyl alcohol was put in a reaction vessel of 300 ml capacity and 10 ml of a 12N hydrochloric acid (0.12 mole as HCl) were added dropwise into the mixture in the vessel followed by further continued agitation of the mixture at room temperature for 15 minutes after completion of the dropwise addition of the hydrochloric acid. Thereafter, 8 ml of a 50% hydrofluoric acid (0.20 mole as HF) were added dropwise into the mixture in the vessel followed by further continued agitation of the mixture at room temperature for 5 minutes after completion of the dropwise addition of the hydrofluoric acid so that the colorless and transparent reaction mixture was converted into a white gel. The gel recovered by filtration on a Buchner funnel was repeatedly washed, first 5 times with water and then 5 times with acetone to give neutral washings of pH 7 as tested with a pH-test paper. The wet gel was dried by heating in a vacuum drying oven at 120 °C under a pressure of 15 mmHg for 2 hours to give 25.4 g of a gelled material containing 4.8% by weight of carbon by the elementary analysis. A strong infrared absorption band assignable to aldehydo groups -CHO was found in the infrared absorption spectrum of the gel at a wave number of 1630 cm⁻¹. Chemical analysis of the gel by the methyl Grignard method indicated that 1 g of the gel contained 4.6 m moles of silanolic hydroxy groups as determined in the form of methane. These analytical results led to a conclusion that the gel could be expressed by the average unit formula

(OCHCH₂CH₂)_{0.09}(HO)_{0.31}SiO_{1.8},

which is referred to as the acetal gel hereinbelow.

### [Immobilization of enzymes]

### 1) Immobilization of aminoacylase

A mixture of 2.0 g of the acetal gel prepared in the above described manner, 10 mg (230 units) of aminoacylase and 67 ml of a phosphate buffer solution having a pH of 7.0 in a reaction vessel of 300 ml capacity was agitated for 3 hours at room temperature and the gel was recovered by filtration on a Buchner funnel and washed twice with a phosphate buffer solution. A spectrophotometric analysis indicated that 76% of the enzyme had been immobilized on the gel corresponding to 86 units of the enzyme per g of the carrier.

### 2) Immobilization of trypsin

The procedure was substantially the same as in the immobilization of aminoacylase described above except that 10 mg of the aminoacylase were replaced with 10 mg (175 units) of trypsin. The result was that 58% of the enzyme was immobilized on the gel corresponding to 51 units of the enzyme per g of the carrier.

### 3) Immobilization of glucoamylase

The procedure was substantially the same as in the immobilization of aminoacylase described above except that 10 mg of the aminoacylase were replaced with 10 mg (124 units) of glucoamylase. The result was that 69% of the enzyme was immobilized on the gel corresponding to 43 units of the enzyme per g of the carrier.

### Example 2.

### [Preparation of an aldehydo-containing organopolysiloxane gel]

Into a reaction vessel of 300 ml capacity were introduced 70 g (0.648 mole) of citronellal CH₃C(CH₃)=CHCH₂CH₂CH(CH₃)CH₂CHO, 25.9 mg (0.05 m mole) of chloroplatinic acid and 70 ml of toluene and the mixture was heated at 60 °C. Then, 119 g (0.972 mole) of trimethoxy silane HSi(OCH₃)₃ were gradually introduced into the mixture kept at the same temperature and the reaction mixture was further agitated at 100 °C for 2 hours after completion of the introduction of the silane compound. After cooling, the reaction mixture was subjected to distillation under reduced pressure to give 34.9 g of a fraction which could be identified to be 7-oxo-1,1,5-trimethylheptyl trimethoxy silane of the formula

(CH₃O)₃SiC(CH₃)₂CH₂CH₂CH₂CH(CH₃)CH₂CHO,

which is referred to as the aldehyde silane hereinbelow. The yield was 0.126 mole corresponding to 20% of the theoretical value.

A mixture composed of 17.9 g (0.065 mole) of the aldehyde eilane, 137 g (0.650 mole) of ethyl silicate and 109 g (2.40 moles) of ethyl alcohol was taken in a reaction vessel of 500 ml capacity and 16 ml of a 12N hydrochloric acid (0.19 mole as HCl) were added dropwise into the mixture in the vessel followed by further continued agitation of the mixture at room temperature for 15 minutes after completion of the dropwise addition of the hydrochloric acid. Thereafter, 13 ml of a 50% hydrofluoric acid (0.325 mole as HF) were added dropwise into the mixture in the vessel followed by further continued agitation of the mixture at room temperature for 10 minutes after completion of the dropwise addition of the hydrofluoric acid so that the colorless and transparent reaction mixture was converted into a white gel. The gelled reaction mixture was processed in the same manner as in Example 1 to give 42.0 g of a dried material containing 12.8% by weight of carbon as determined by the elementary analysis. A strong infrared absorption band assignable to aldehydo groups -CHO was found in the infrared absorption spectrum of the gel at a wave number of 1630 cm⁻¹. Chemical analysis of the gel by the methyl Grignard method indicated that 1 g of the gel contained 4.5 m moles of silanolic hydroxy groups as determined in the form of methane. These analytical results led to a conclusion that the gel could be expressed by the average unit formula

[OCHCH₂CH(CH₃)CH₂CH₂CH₂C(CH₃)₂]_{0.08}(HO)_{0.35}SiO_{1.79},

which is referred to as the aldehyde gel I hereinbelow.

### [Immobilization of aminoacylase]

A mixture of 2.0 g of the aldehyde gel I prepared in the above described manner, 10mg (230 units) of aminoacylase and 67 ml of a phosphate buffer solution having a pH of 7.0 in a reaction vessel of 300 ml capacity was agitated for 3 hours at room temperature and the mixture was processed in the same manner as in Example 1. A spectrophotometric analysis indicated that 60% of the enzyme had been immobilized on the gel corresponding to 69 units of the enzyme per g of the carrier.

### Example 3.

A mixture composed of 7.0 g (0.065 mole) of the aldehyde silane prepared in Example 2, 90 g (0.600 mole) of methyl trichlorosilane and 109 g (2.40 moles) of ethyl alcohol was taken in a reaction vessel of 500 ml capacity and 16 ml of a 12N hydrochloric acid (0.19 mole as HCl) and then 13 ml of a 50% hydrofluoric acid (0.325 mole as HF) were added dropwise successively into the mixture. The mixture was subsequently processed in the same manner as in Example 1 to give 41.0 g of a gel containing 25.8% by weight of carbon and 8.0% by weight of chlorine as determined by the elementary analysis. A strong infrared absorption band assignable to aldehydo groups -CHO was found in the infrared absorption spectrum of the gel at a wave number of 1630 cm⁻¹. Chemical analysis of the gel by the methyl Grignard method indicated that 1 g of the gel contained 1.1 m moles of silanolic hydroxy groups as determined in the form of methane. These analytical results led to a conclusion that the gel could be expressed by the average unit formula

[OHCCH₂CH(CH₃)CH₂CH₂CH₂C(CH₃)₂]_{0.10}(CH₃)_{0.90} (OH)_{0.20}(Cl)_{0.20}SiO_{1.30},

which is referred to as the aldehyde gel II hereinbelow.

A mixture of 2.0 g of the aldehyde gel II prepared in the above described manner, 10 mg (230 units) of aminoacylase and 67 ml of a phosphate buffer solution having a pH of 7.0 in a reaction vessel of 300 ml capacity was agitated for 3 hours at room temperature and the mixture was subsequently processed in the same manner as in Example 1. A spectrophotometric analysis indicated that 44% of the enzyme had been immobilized on the gel corresponding to 51 units of the enzyme per g of the carrier.

### Comparative Example 1.

Into a reaction vessel of 1 liter capacity were introduced 312 g (1.50 moles) of ethyl silicate and 250 g (5.4 moles) of ethyl alcohol and 37.5 ml of a 12N hydrochloric acid (0.45 mole as HCl) were added dropwise into the mixture in the vessel followed by agitation of the mixture at room temperature for 15 minutes. Then, 30 g of a 50% hydrofluoric acid (0.75 mole as HF) were added dropwise into the mixture over a period of 5 minutes so that the clear and colorless reaction mixture was suddenly converted into a white gel at a moment 30 seconds after completion of the dropwise addition of the hydrofluoric acid. The gelled reaction mixture was heated at 80 °C for 2 days and then dried by heating in a vacuum drying oven at 80 °C for 2 hours under a pressure of 2 mmHg to give 86.4 g (1.44 moles) of a glassy porous silica body corresponding to a yield of 96% of the theoretical value.

A 5.0 g (0.083 mole) portion of this porous silica was put in a reaction vessel of 300 ml capacity and an ethyl alcohol solution containing 5.0 g (0.018 mole) of the aldehyde silane prepared in Example 2 and having a pH of 3.5 as adjusted with a diluted hydrochloric acid was added thereto. The mixture was heated at 100 °C for 2 hours and then filtered with a Buchner funnel to recover the porous silica which was washed twice with water and then twice with acetone followed by drying in a vacuum drying oven at 120 °C for 2 hours under a pressure of 2 mmHg to give 4.6 g of dried silica having aldehydo groups.

Immobilization of aminoacylase on the thus obtained porous silica was undertaken in the same manner as in Example 1 to find that 32% of the enzyme was immobilized on the silica corresponding to 74 units of the enzyme per g of the carrier.

### Comparative Example 2.

A 20 g (0.33 mole) portion of the glassy porous silica prepared in Comparative Example 1 described above was put in a reaction vessel and 400 ml of an aqueous solution containing 29 g (0.165 mole) of 3-aminopropyl triethoxy silane and having a pH of 3.5 as adjusted with a diluted hydrochloric acid were added thereto. The mixture was then heated at 100 °C for 2 hours and filtered with a Buchner funnel followed by processing in the same manner as in Comparative Example 1 to give 21 g of silica treated with the amino-containing silane.

The silica after the aminosilane treatment was further treated with the aldehyde silane. Thus, 11.5 g of the silica and 200 ml of a 0.1M aqueous solution of sodium phosphate containing 5% by weight of glutaraldehyde were taken in a reaction vessel of 300 ml capacity and the mixture was aged by keeping for 24 hours at room temperature. Thereafter, the silica was treated in the same manner as in Comparative Example 1 to give 12.5 g of a silica material containing aldehydo groups and 3.8% by weight of carbon as determined by the elementary analysis. A strong infrared absorption band assignable to aldehydo groups -CHO was found in the infrared absorption spectrum of the silica at a wave number of 1630 cm⁻¹. Chemical analysis of the gel by the methyl Grignard method indicated that 1 g of the silica contained 4.1 m moles of silanolic hydroxy groups as determined in the form of methane. These analytical results led to a conclusion that the gel could be expressed by the average unit formula

(OCHCH₂CH₂CH₂CH=NCH₂CH₂)_{0.03}(HO)_{0.27}SiO_{1.85},

which is referred to as the aldehyde gel III hereinbelow.

The thus obtained aldehyde gel III was used as a carrier for the immobilization of aminoacylase in the same manner as in Example 1 to find that only 22% of the enzyme was immobilized on the silica corresponding to 25 units of the enzyme per g of the carrier.

## Claims

1. A method of preparing an organopolysiloxane gel of a chemical composition represented by the average unit formula 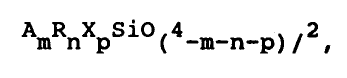 in which A which is a monovalent hydrocarbon group having at least one aldehydo group -CHO or an acetal group -CH(OR¹)₂, R¹ being an alkyl group, R is a monovalent hydrocarbon group having 1 to 18 carbon atoms, X is a hydroxy group or a silicon-bonded hydrolyzable group, the subscript m is a positive number in the range from 0.01 to 1, the subscript n is zero or a positive number smaller than 1 with the proviso that m+n is a positive number not exceeding 1 and the subscript p is a positive number smaller than 0.5 which comprises hydrolyzing an organosilane compound having a silicon-bonded group A which is a monovalent hydrocarbon group having at least one aldehydo group -CHO or an acetal group -CH(OR¹)₂, R¹ being an alkyl group, and a silicon-bonded hydrolyzable group or cohydrolyzing a silane mixture comprising such an organosilane compound and another hydrolyzable organosilane compound in the presence of hydrofluoric acid.

2. A method as claimed in claim 1 wherein A is
-CH₂CH₂CHO;
-CHMeCH₂CHO;
-CH₂CH₂CH(OMe)₂;
-CH₂CH₂CH(OEt)₂;
-CH₂CH(OEt)₂;
-CMe₂(CH₂)₃CHMeCH₂CHO;
-CMe₂(CH₂)₃CHMeCH(OMe)₂;
-CHPr(CH₂)₂CHMeCH₂CHO;
-CHPr(CH₂)₂CHMeCH₂CH(OMe)₂;
-(CH₂)₁₀-CHO; or
-(CH₂)₁₀-CH(OMe)₂

3. A method as claimed in claim 1 wherein A is -CH₂CH₂CHO, -C(CH₃)₂ (CH₂)₃CH(CH₃)CH₂CHO or -CH₂CH₂CH(OEt)₂.

4. A method as claimed in any one of the preceding claims wherein R is an alkyl, alkenyl, aryl or cycloalkyl group.

5. A method as claimed in any one of the preceding claims wherein X is silicon-bonded alkoxy or acyloxy.

## Patentansprüche

1. Verfahren zur Herstellung von Organopolysiloxan-Gelen, die hinsichtlich ihrer chemischen Zusammensetzung aus durchschnittlichen Einheiten der Formel 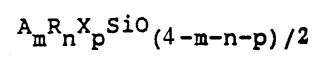 aufgebaut sind,
worin bedeuten:
A eine einwertige Kohlenwasserstoffgruppe, die mindestens eine Aldehydgruppe -CHO oder mindestens eine Acetalgruppe -CH(OR¹)₂ aufweist, wobei R¹ eine Alkylgruppe darstellt,
R eine einwertige Kohlenwasserstoffgruppe mit 1 bis 18 C-Atomen,
X Hydroxy oder eine an Silicium gebundene hydrolysierbare Gruppe,
m eine positive Zahl im Bereich von 0,01 bis 1,
n 0 oder eine positive Zahl < 1 mit der Maßgabe, daß m+n eine positive Zahl ≦ 1 ist,
und
p eine positive Zahl < 0,5,
das die Hydrolyse einer Organosilanverbindung, die eine an Silicium gebundene Gruppe A aufweist, die eine einwertige Kohlenwasserstoffgruppe darstellt, die mindestens eine Aldehydgruppe -CHO oder mindestens eine Acetylgruppe -CH(OR¹)₂, wobei R¹ eine Alkylgruppe darstellt, und eine an Silicium gebundene hydrolysierbare Gruppe aufweist, oder die Cohydrolyse eines Silangemischs, das eine derartige Organosilanverbindung sowie eine weitere hydrolysierbare Organosilanverbindung enthält, in Gegenwart von Fluorwasserstoffsäure umfaßt.

2. Verfahren nach Anspruch 1, wobei A
-CH₂CH₂CHO,
-CHMeCH₂CHO,
-CH₂CH₂CH(OMe)₂,
-CH₂CH₂CH(OEt)₂,
-CH₂CH(OEt)₂,
-CMe₂(CH₂)₃CHMeCH₂CHO,
-CMe₂(CH₂)₃CHMeCH(OMe)₂,
-CHPr(CH₂)₂CHMeCH₂CHO,
-CHPr(CH₂)₂CHMeCH₂CH(OMe)₂,
-(CH₂)₁₀-CHO oder
-(CH₂)₁₀-CH(OMe)₂
ist.

3. Verfahren nach Anspruch 1, wobei A -CH₂CH₂CHO, -C(CH₃)₂(CH₂)₃CH(CH₃)CH₂CHO oder -CH₂CH₂CH(OEt)₂ ist.

4. Verfahren nach einem der vorhergehenden Ansprüche, wobei R Alkyl, Alkenyl, Aryl oder Cycloalkyl ist.

5. Verfahren nach einem der vorhergehenden Ansprüche, wobei X an Silicium gebundendes Alkoxy oder Acyloxy ist.

## Revendications

1. Méthode de préparation d'un gel d'organopolysiloxane ayant la composition chimique représentée par la formule unitaire moyenne 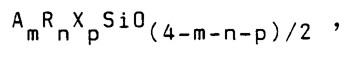 où A est un groupe hydrocarbure monovalent ayant au moins un groupe aldéhyde -CHO ou un groupe acétal -CH(OR¹)₂, R¹ étant un groupe alkyle, R est un groupe hydrocarbure monovalent ayant 1 à 18 atomes de carbone, X est un groupe hydroxy ou un groupe hydrolysable lié au silicium, l'indice m est un nombre positif compris entre 0,01 et 1, l'indice n est zéro ou un nombre positif plus petit que 1, à condition que m+n soit un nombre positif ne dépassant pas 1 et l'indice p est un nombre positif plus petit que 0,5, qui comprend l'hydrolyse d'un composé d'organosilane ayant un groupe A lié au silicium qui est un groupe hydrocarbure monovalent ayant au moins un groupe aldéhyde -CHO ou un groupe acétal -CH(OR¹)₂ , R¹ étant un groupe alkyle, et un groupe hydrolysable lié au silicium ou la co-hydrolyse d'un mélange de silanes comprenant un tel composé d'organosilane et autre composé d'organosilane hydrolysable, en présence d'acide fluorhydrique.

2. Méthode selon la revendication 1, où A est
-CH₂CH₂CHO ;
-CHMeCH₂CHO ;
-CH₂CH₂CH(OMe)₂ ;
-CH₂CH₂CH(OEt)₂ ;
-CH₂CH(OEt)₂ ;
-CMe₂(CH₂)₃CHMeCH₂CHO ;
-CMe₂(CH₂)₃CHMeCH(OMe)₂ ;
-CHPr(CH₂)₂CHMeCH₂CHO ;
-CHPr(CH₂)₂CHMeCH₂CH(OMe)₂ ;
-(CH₂)₁₀-CHO ; ou
-(CH₂)₁₀-CH(OMe)₂

3. Méthode selon la revendication 1, où A est -CH₂CH₂CHO, -C(CH₃)₂ (CH₂)₃CH(CH₃)CH₂CHO ou -CH₂CH₂CH(OEt)₂.

4. Méthode selon l'une quelconque des revendications précédentes où R est un groupe alkyle, alkényle, aryle ou cycloalkyle.

5. Méthode selon l'une quelconque des revendications précédentes, où X est acyloxy ou alcoxy lié au silicium.
